(19) Europäisches Patentamt
European Patent Office
Office européen des brevets

(11) **EP 2 719 260 B1**

(12) **FASCICULE DE BREVET EUROPEEN**

(45) Date de publication et mention de la délivrance du brevet:
**01.04.2020 Bulletin 2020/14**

(21) Numéro de dépôt: **12731554.7**

(22) Date de dépôt: **08.06.2012**

(51) Int Cl.:
*H05G 2/00* *(2006.01)*    *H05H 1/22* *(2006.01)*

(86) Numéro de dépôt international:
**PCT/FR2012/051297**

(87) Numéro de publication internationale:
**WO 2012/168670 (13.12.2012 Gazette 2012/50)**

(54) **PROCÉDÉ ET AGENCEMENT POUR ENGENDRER UN JET DE FLUIDE, PROCÉDÉ ET SYSTÈME DE TRANSFORMATION DU JET EN UN PLASMA ET APPLICATIONS DE CE SYSTÈME**

VERFAHREN UND ANORDNUNG ZUR ERZEUGUNG EINES FLÜSSIGKEITSSTRAHLS, VERFAHREN UND SYSTEM ZUR TRANSFORMATION DES STRAHLS IN PLASMA UND ANWENDUNGEN DIESES SYSTEMS

METHOD AND ARRANGEMENT FOR GENERATING A JET OF FLUID, METHOD AND SYSTEM FOR TRANSFORMING THE JET INTO PLASMA, AND USES OF SAID SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.06.2011 FR 1155056**

(43) Date de publication de la demande:
**16.04.2014 Bulletin 2014/16**

(73) Titulaires:
• **Ecole Polytechnique**
  **91120 Palaiseau (FR)**
• **Centre National de la Recherche Scientifique**
  **75016 Paris (FR)**

(72) Inventeurs:
• **SYLLA, Soriba Franciszek (François)**
  **F-94230 Cachan (FR)**
• **MALKA, Victor, Armand**
  **F-75005 Paris (FR)**
• **FLACCO, Alessandro, Federico**
  **92120 MONTROUGE (FR)**
• **VELTCHEVA, Mina Ivanova**
  **F-75013 Paris (FR)**
• **KAHALY, Subhendu**
  **F-91140 Villebon-sur-yvette (FR)**

(74) Mandataire: **Berger, Helmut**
  **Cabinet Weinstein**
  **176 avenue Charles de Gaulle**
  **92200 Neuilly sur Seine (FR)**

(56) Documents cités:
  **DE-C1- 19 503 549    KR-U- 20100 012 708**
  **US-A- 4 589 123    US-A- 5 577 092**

## Description

L'invention concerne un procédé et un agencement pour générer un jet de fluide de forte densité atomique ainsi qu'un procédé et un système de transformation d'un tel jet de fluide en un plasma à l'aide d'un faisceau laser, ainsi que des procédés d'application du système de transformation.

Des procédés et systèmes de ce type sont déjà connus, mais ne permettent pas d'obtenir des jets de fluide d'une cadence et de densité atomique suffisantes pour assurer une exploitation efficace des possibilités techniques potentielles de ces jets et, notamment de celles qui pourraient résulter des interactions de ces jets de fluide avec des faisceaux laser.

L'invention a pour but de pallier cet inconvénient.

Pour atteindre ce but, le procédé pour générer le jet de fluide de forte densité est caractérisé en ce qu'il comprend l'opération de créer à l'aide d'une électrovanne rapide haute pression, suivie d'une tuyère montée à l'ouverture de sortie, un jet de fluide pulsé de dimensions submillimétriques et d'une densité atomique supérieure à $10^{20}$ cm$^{-3}$.

Selon une caractéristique de l'invention, le procédé est caractérisé en ce que la densité atomique du jet de fluide présente une valeur de $10^{21}$ cm$^{-3}$ ou plus.

Selon une autre caractéristique de l'invention, le procédé est caractérisé en ce que le fluide est un gaz tel que de l'hélium.

Selon encore une autre caractéristique de l'invention, le procédé est caractérisé en ce que le fluide est biphasique tel qu'un jet d'agrégat.

Selon encore une autre caractéristique de l'invention, le procédé est caractérisé en ce que le fluide est un liquide tel que de l'eau.

Selon encore une autre caractéristique de l'invention, un agencement générateur d'un jet de fluide de forte densité atomique, pour la mise en œuvre du procédé ci-dessus décrit est divulgué à la revendication 6.

Selon encore une autre caractéristique de l'invention, l'agencement est caractérisé en ce que l'électrovanne comporte un solénoïde et un élément mobile d'ouverture et de fermeture rapide du canal de passage du fluide à travers l'électrovanne, qui est déplaçable latéralement à ce canal, dans sa position d'ouverture du canal, sous l'effet du champ magnétique produit par le solénoïde et dont le retour dans sa position de fermeture du canal se produit sous l'effet du fluide sous pression.

Selon encore une autre caractéristique de l'invention, l'agencement est caractérisé en ce que l'entrefer autour du canal comporte au niveau de l'élément mobile une asymétrie ayant pour conséquence la création d'une composante de champ magnétique orientée perpendiculairement au canal et destinée à provoquer le déplacement de l'élément mobile vers sa position d'ouverture du canal.

L'invention concerne également un procédé pour créer un plasma, qui est caractérisé en ce que l'on utilise un jet de fluide et en ce que l'on fait agir sur le jet sortant de la tuyère un laser dont le faisceau est orienté perpendiculairement au jet, à l'intérieur d'une enceinte à vide.

Selon une caractéristique de l'invention, le procédé pour créer un plasma est caractérisé en ce que l'on crée un jet de fluide pulsé d'une forte densité atomique, mais d'une largeur suffisamment faible pour que l'on puisse utiliser pour la création du vide dans l'enceinte une pompe à très haute vitesse, telle qu'une pompe de type turbo-moléculaire.

Selon une autre caractéristique de l'invention, le procédé pour créer un plasma est caractérisé en ce que l'on crée un vide de l'ordre de $10^{-7}$ bar à l'intérieur de l'enceinte.

L'invention concerne également un système générateur d'un plasma pour la mise en œuvre du procédé pour créer un plasma, qui est caractérisé en ce qu'il comprend une source d'un fluide sous pression, un agencement générateur d'un jet de fluide, un laser dont le faisceau agit sur le jet de fluide à la sortie de la tuyère, une enceinte à vide dans laquelle est disposé l'agencement générateur du jet et dans lequel a lieu l'impact du laser sur le jet de fluide et en ce qu'il comporte une pompe de création du vide à l'intérieur de l'enceinte, qui est du type à très haute vitesse telle qu'une pompe turbo-moléculaire.

L'invention concerne également un procédé d'application du système générateur d'un plasma, qui est caractérisé en ce que le système est utilisé pour produire des faisceaux d'ions de spectre fin à haute cadence dans la direction du laser et d'émission d'ions de spectre large à haute cadence dans la direction perpendiculaire au laser.

Selon une caractéristique de l'invention, ce procédé d'application du système est caractérisé en ce que l'on utilise un faisceau laser intense composé d'une ou de plusieurs impulsions courtes de l'ordre de quelques picosecondes et d'un écart entre les impulsions de quelques picosecondes, focalise ce faisceau laser sur un jet de fluide à haute cadence et de densité sur-critique pour ce laser.

Selon une autre caractéristique de l'invention, le procédé d'application du système est caractérisé en ce que l'on filtre le faisceau d'ions à haute cadence sortant du plasma dans l'axe laser en utilisant avantageusement un filtre mobile tel qu'une feuille en aluminium de quelques dizaines à centaines de micromètres pour permettre de rafraîchir la surface que le faisceau endommage à chaque impact.

L'invention concerne encore un procédé d'application du système qui est caractérisé en ce que l'on utilise le système pour produire un faisceau à haute cadence.

Selon encore une autre caractéristique de l'invention, le procédé d'application du système est caractérisé en ce que l'on focalise une impulsion laser intense dans un jet de fluide haute cadence et de forte densité telle qu'une densité de l'ordre de $10^{19}$ atomes/cm$^3$ pour produire un faisceau d'électrons faiblement divergent et d'une courte durée, refocalise spatialement ce faisceau et fait passer le faisceau refocalisé à travers un onduleur.

Selon encore une autre caractéristique de l'invention, le procédé d'application du système est caractérisé en ce que l'on fait passer le faisceau ayant traversé l'onduleur par un dispositif qui fait dévier les électrons et permet d'obtenir un faisceau seulement de rayons X.

Selon encore une autre caractéristique de l'invention, le procédé d'application du système est caractérisé en ce qu'on utilise un onduleur constitué d'une succession d'aimants de polarité alternés de sorte que les électrons ondulent au passage de l'onduleur et rayonnent des rayons X vers l'avant dans la direction du faisceau.

Selon encore une autre caractéristique de l'invention, le procédé d'application du système est caractérisé en ce que l'on fait varier la gamme spectrale des rayons X par variation de la période des aimants alternés et leur puissance.

L'invention concerne encore un procédé d'application du système, qui est caractérisé en ce que l'on utilise ce système pour la production de faisceaux gamma à haute cadence.

Selon une autre caractéristique de l'invention, ce procédé d'application du système est caractérisé en ce que l'on produit les faisceaux gamma à haute cadence par conversion d'un faisceau d'électrons à haute cadence, obtenu à partir du plasma produit par interaction d'une impulsion laser intense avec un jet de gaz haute cadence et de forte densité telle qu'une densité de l'ordre de $10^{19}$ atomes/cm$^3$.

Selon encore une autre caractéristique de l'invention, le procédé d'application du système est caractérisé en ce que l'on focalise l'impulsion laser intense dans le jet de gaz et utilise une optique de focalisation de focal adapté à la longueur du jet pour que le laser s'y propage de façon régulière, sans filamentation.

Selon encore une autre caractéristique de l'invention, le procédé d'application du système est caractérisé en ce que l'on fait impacter le faisceau d'électrons un convertisseur mobile pour provoquer l'émission de rayons gamma dus au freinage des électrons par collision sur les atomes du convertisseur.

Selon encore une autre caractéristique de l'invention, le procédé d'application du système est caractérisé en ce que l'on utilise un dispositif tel qu'un aimant bipolaire pour faire dévier les électrons et l'obtention d'un faisceau de rayons gamma à haute cadence.

L'invention concerne encore un procédé d'application du système, qui est caractérisé en ce que le système est utilisé pour réaliser un nettoyage temporel des impulsions intenses du laser à l'aide du jet de fluide.

Selon une caractéristique de l'invention, le procédé est caractérisé en ce que l'impulsion laser à nettoyer est amenée à être incidente sur un jet de fluide à densité sur-critique pour la longueur d'onde du laser de façon que la lumière parasite de l'impulsion ne produise pas un claquage du plasma et soit translucide à cette lumière et que le claquage ne soit produit que par la partie utile de l'impulsion laser qui est alors réfléchie par le jet de fluide.

L'invention concerne encore un procédé d'application du système, qui est caractérisé en ce que l'on utilise ce système pour réaliser une compression temporelle et spatiale des impulsions laser.

Selon une caractéristique de l'invention, le procédé est caractérisé en ce qu'une impulsion laser intense à comprimer est focalisée dans le jet de gaz haute cadence et de forte densité telle qu'une densité de l'ordre de $10^{19}$ atomes/cm$^3$ et fait en sorte que les petites longueurs d'onde du spectre de l'impulsion arrivent sur le plasma avant les grandes longueurs d'onde de façon que la propagation moins vite des petites longueurs d'onde dans le plasma provoque une recompression de l'impulsion dans le temps. Le document US5,577,092 divulgue un exemple de dispositif et de procédé de production d'un jet de fluide sous haute pression.

L'invention sera mieux comprise et d'autres buts, détails et avantages de celle-ci apparaitront plus clairement au cours de la description qui va suivre, faite en référence aux dessins annexés représentant un mode de réalisation de l'invention, et dans lesquels :

- la figure 1 est une vue schématique d'un agencement selon l'invention ;
- les figures 2A et 2B sont des vues respectivement en perspective et de coupe axiale d'un ensemble électrovanne et tuyère de l'agencement selon la figure 1 ;
- les figures 3A et 3B sont deux vues schématiques en coupe axiale d'une autre version d'une électrovanne pouvant être utilisée dans une installation selon la figure 1, pour expliquer le principe de fonctionnement d'une telle électrovanne ;
- les figures 4A, 4B et 4C sont trois vues d'une tuyère selon l'invention, la figure 4A étant une vue de dessus, la figure 4B une vue en coupe axiale selon la ligne IV-IV de la figure 4A et la figure 4C est une vue, à plus grande échelle, du détail entouré d'un cercle sur la figure 4B ;
- la figure 5 illustre sous forme d'une courbe la densité D du jet de fluide en fonction de la pression P produite par le surpresseur ;
- la figure 6 montre sous forme d'une courbe la densité D du fluide en fonction du rayon R ;
- la figure 7 est une vue schématique illustrant une première application de l'invention destinée à la production de faisceaux d'ions de spectre fin à haute cadence dans la direction du laser et d'émissions d'ions de spectre large à haute cadence dans la direction perpendiculaire au laser ;
- la figure 8 est une vue schématique illustrant une deuxième application de l'invention permettant la

5        EP 2 719 260 B1        6

production de faisceaux X à haute cadence ;

- la figure 9 est une vue schématique illustrant une troisième application de l'invention, permettant la production de faisceaux gamma à haute cadence ; et
- la figure 10 est une vue schématique illustrant encore une autre application de l'invention permettant le nettoyage temporel d'une impulsion laser intense.

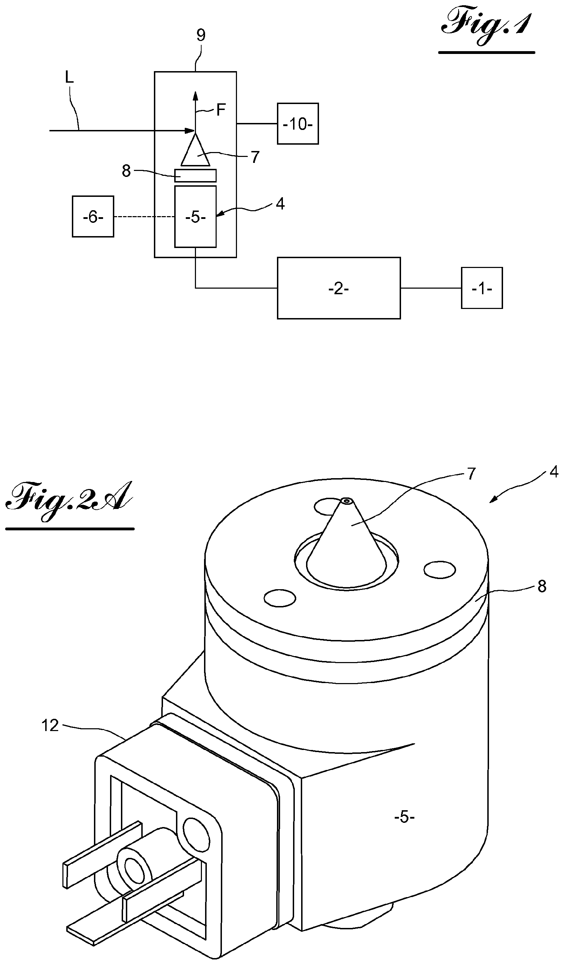

La figure 1 illustre, à titre d'exemple, un agencement générateur d'un plasma par interaction d'un jet de fluide millimétrique ou submillimétrique de haute cadence et de forte densité pouvant être supérieure à $10^{19}$ atomes/cm$^3$ et aller jusque $10^{21}$ atomes/cm$^3$ et plus. Le système comporte essentiellement une source 1 d'un fluide tel qu'un gaz, par exemple de l'hélium, ou d'un liquide tel que de l'eau, qui est transmis à un surpresseur 2 qui, à son tour, transmet le fluide fortement comprimé à un dispositif 4 générateur du jet de fluide pulsé indiqué par la flèche F et formé par une succession d'impulsions d'une durée de quelques microsecondes et de la densité susmentionnée, supérieure à $10^{19}$ atomes/cm$^3$.

Le dispositif générateur 4 comporte une électrovanne 5 dont l'élément mobile présente une très faible inertie et qui a donc un temps de réponse très faible. A titre d'exemple, l'ouverture et la fermeture de l'électrovanne peut se faire en moins de trois millisecondes, c'est-à-dire à une cadence d'environ 300 Hz. Cette électrovanne qui sera décrite plus en détail plus loin est commandée par un dispositif de commande électrique 6. Une tuyère 7 est montée sur la sortie de l'électrovanne par l'intermédiaire d'un adaptateur 8 qui est par exemple vissé sur la sortie de l'électrovanne. Le dispositif générateur 4 est enfermé dans une enceinte étanche 9 dans laquelle on crée un vide de par exemple $10^{-7}$ bars à l'aide d'une pompe à très haute vitesse, par exemple du type turbomoléculaire 10. Un vide important de préférence de cet ordre ou inférieur est souhaitable sinon nécessaire pour éviter les interactions entre le laser et des molécules de l'air restant à l'intérieur de l'enceinte.

Le dispositif générateur 4, grâce à la tuyère 7, est adapté de façon à pouvoir accélérer et structurer l'écoulement de fluide, lorsque l'électrovanne est actionnée, afin que cet écoulement présente en sortie de tuyère les propriétés désirées par l'opérateur.

Si le jet de fluide sortant de la tuyère 7 doit être transformé en un plasma, on fait agir un faisceau laser symbolisé par la flèche L sur le jet de fluide matérialisé par la flèche F, qui sort de la tuyère.

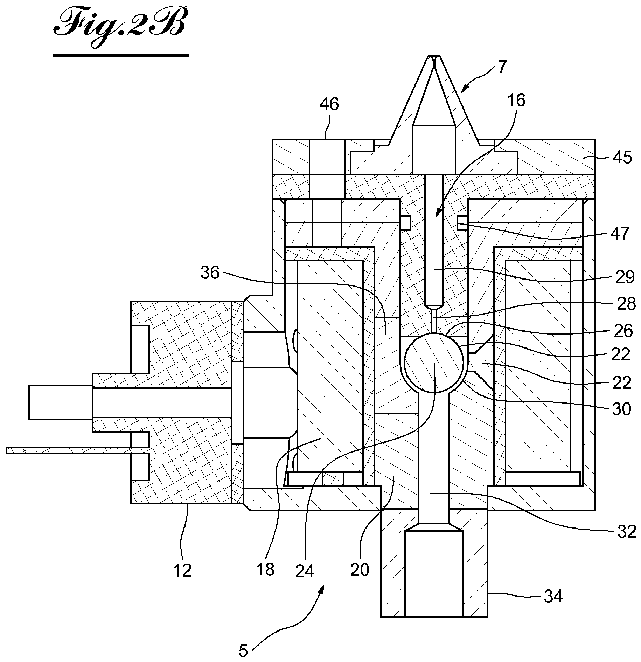

Les figures 2A et 2B illustrent, à titre d'exemple, un dispositif générateur de jet de fluide de haute cadence et de forte densité, tel qu'indiqué en 4 sur la figure 1. Les figures 2A et 2B illustrent en 4 l'électrovanne, en 8 l'adaptateur et en 7 la tuyère. La référence 12 indique le connecteur au dispositif de commande de l'électrovanne.

L'électrovanne 5 comporte un corps cylindrique qui est axialement traversé, dans son centre, par un canal 16 et loge un solénoïde 18 qui est disposé coaxialement autour du canal en laissant subsister entre ce canal et le solénoïde une paroi 20 en un matériau magnétisable et à travers lequel passent donc les lignes du champ magnétique créé par le solénoïde lorsqu'il est excité, c'est-à-dire parcouru par un courant électrique. Le connecteur 12 est réalisé sous forme d'un embout radial au corps cylindrique.

Comme le montre la figure 2B, la paroi 20 délimite dans le corps 14 une chambre de vanne 22 qui loge, dans l'exemple représenté, une bille 24 en un matériau ferromagnétique et qui constitue l'élément mobile d'ouverture et de fermeture du canal axial de la vanne. La chambre est suffisamment large pour permettre un mouvement latéral de l'élément mobile 24, perpendiculairement à l'axe du canal.

La chambre est donc délimitée latéralement par la paroi 20 et en haut sur la figure, perpendiculairement à la paroi 20 par une paroi sensiblement plane 26 au centre de laquelle s'ouvre le canal 16 dans la chambre 22. Cette partie du canal notée 28 est plus étroite que la partie de canal supérieure 29 qui communique avec la tuyère 7. A l'état de repos, lorsque l'électrovanne n'est pas excitée, l'élément mobile 24 obture le canal 28 dont la zone d'ouverture dans la chambre 22 est, à cette fin, configurée pour constituer le siège d'étanchéité de cet élément 24.

Au côté axialement opposé à la paroi transversale 26, la chambre 22 présente une face concave 30 en forme d'une calotte sphérique, complémentaire à l'élément 24, et au centre de laquelle débouche le canal 16, ici formé par une partie de canal plus large 32 qui est destinée à communiquer avec le surpresseur 2. A cette fin, l'électrovanne comporte un embout de connexion axial 34.

L'élément en forme de bille 24 est déplaçable latéralement entre sa position centrale sur le siège 26 dans laquelle l'élément ferme le canal 16 en obturant la portion de canal 28 et une position latéralement décalée du siège dans laquelle le canal est ouvert et permet l'écoulement du fluide sous pression en provenance du surpresseur 2, à la tuyère 7.

Ce déplacement latéral d'ouverture du canal 16 est obtenu par excitation du solénoïde 18 sous l'effet du champ magnétique produit par celui-ci. A cette fin, le champ magnétique doit présenter au niveau de l'élément mobile 24 une composante qui est orientée perpendiculairement au canal. Cette composante est obtenue en créant une irrégularité dans la partie d'entrefer de la paroi 20 au niveau de la bille 24. A cette fin, cette paroi comporte donc au niveau de la bille des inserts 36 et 37, qui sont disposés sur le dessin respectivement à gauche et à droite de la bille 24. Ces inserts ont une sensibilité magnétique différente du restant de la paroi 20. En donnant à ces inserts des formes différentes, comme l'illustre la figure, on crée dans le circuit magnétique une asymétrie qui génère la composante perpendiculaire du champ magnétique, qui provoque le déplacement latéral de la bille. A la fin de l'excitation du solénoïde, la bille revient dans sa position centrale dans la chambre.

4

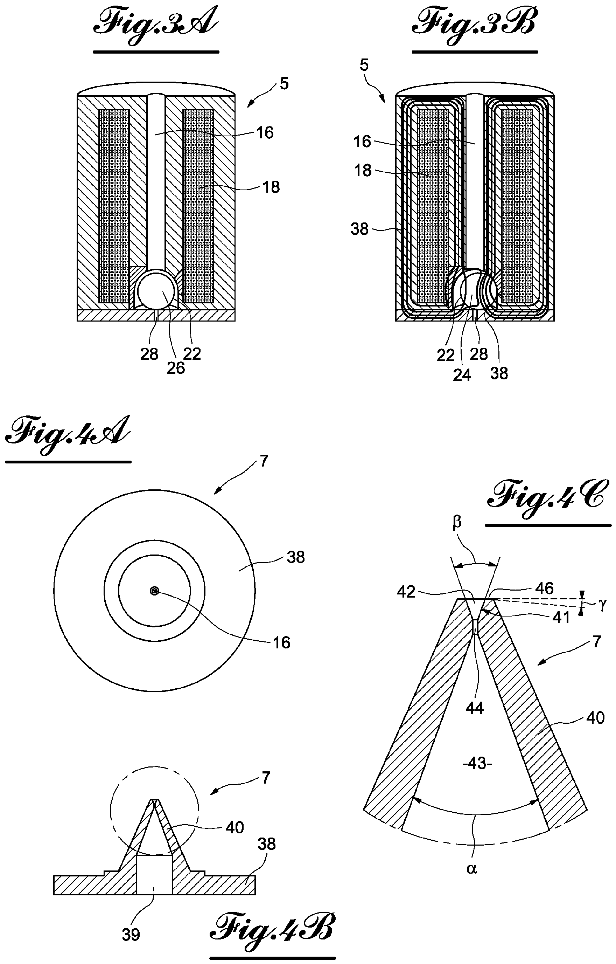

Les figures 3A, 3B, bien que montrant schématiquement une autre version d'une électrovanne, cependant du même type de structure et de fonctionnement, illustrent ce qui vient d'être écrit. En effet, la figure 3A montre la bille 24 de fermeture du canal 16 dans sa position d'obturation du canal et la figure 3B montre cette bille dans sa position latéralement décalée d'ouverture du canal, sous l'effet du champ magnétique produit par le solénoïde 18. La figure 3B illustre en 38 les lignes du champ magnétique qui présente au niveau de la bille des excroissances latérales dues à l'asymétrie de l'entrefer créée par les inserts 36, 37. Par conséquent, le champ magnétique au niveau de la bille 24 présente une composante perpendiculaire au canal 16 qui engendre donc la force de déplacement latéral de cette bille.

Ainsi, lorsque le solénoïde est excité, le corps 24 bascule de sa position de repos vers un côté du siège 26, en raison de l'asymétrie du champ magnétique, ce qui permet au fluide de circuler librement à travers le canal 16. A l'arrêt de l'excitation, le corps 24 rebascule vers sa position de repos à cause de la friction du fluide circulant, ce qui assure la fermeture du canal 16.

Le système de basculement assure une réponse rapide. A titre d'exemple, l'ouverture et la fermeture de l'électrovanne peut se faire en moins de 3 millisecondes, donc à une cadence d'environ 300 Hz, et la force magnétique développée par le solénoïde permet le mouvement du corps mobile, c'est-à-dire de la bille 24 pressée contre son siège par un fluide par exemple de l'ordre de 400 bar à température ambiante, qui pourrait monter jusqu'à 1000 bar. L'alimentation électrique pilotable permet de délivrer la puissance électrique nécessaire pendant une durée ajustable par l'opérateur, à titre d'exemple à partir d'une picoseconde. Cela permet de faire varier finement la période d'oscillation de la bille 24 et donc la quantité de fluide qui passe dans l'électrovanne, c'est-à-dire le débit, et au final la densité, que peut délivrer l'électrovanne à la sortie dans la tuyère 7. De même, pour une période d'oscillation fixe, on peut faire varier la pression du fluide en entrée de l'électrovanne et donc faire varier le débit en sortie.

Les figures 4A à 4C montrent la tuyère de sortie 7 qui est positionnée sur la sortie de l'électrovanne, par l'intermédiaire de l'adaptateur 8 fixé sur l'électrovanne. Celui-ci se visse sur la sortie de la vanne.

La tuyère 7 est fixée sur l'adaptateur 8 et maintenue solidaire de celui-ci par un capuchon 45 que l'on fixe sur l'électrovanne par des vis dont les trous de passage sont indiqués en 45. Il est à noter qu'un. joint circulaire en caoutchouc 45 entouré d'une bague métallique est prévu pour assurer l'étanchéité du circuit à haute pression. La bague métallique assure l'écrasement homogène du joint contre l'adaptateur et évite le déplacement du joint quand la haute pression est appliquée.

Dans l'exemple représenté, la tuyère comporte une base 38 en forme d'un disque dont la partie centrale est percée par un office central 39 de passage du fluide sous pression, qui est en alignement avec le canal 16 traversant l'électrovanne, et une partie tubulaire conique 40 qui se rétrécit en direction de l'orifice de sortie 41 dont la partie de pointe communique avec l'intérieur de l'espace interne conique 43 de la tuyère par une courte portion cylindrique 44. Ainsi, la tuyère est du type convergent/divergent produisant un écoulement supersonique. Bien entendu, des tuyères d'autre type pourraient être envisagées, par exemple une tuyère à orifice de sortie cylindrique pouvant produire un écoulement de fluide sonique ou subonique.

Concernant la tuyère des figures 4, à propos de son dimensionnement, à titre d'exemple, les angles $\alpha$ de convergence et $\beta$ de divergence pourraient être tous les deux de l'ordre de 40° et le diamètre de la petite portion cylindrique 44 entre les portions de convergence et de divergence pourrait avoir un diamètre de 0,1 mm. La partie conique divergente 42 pourrait présenter à sa sortie un diamètre de 0,4 mm.

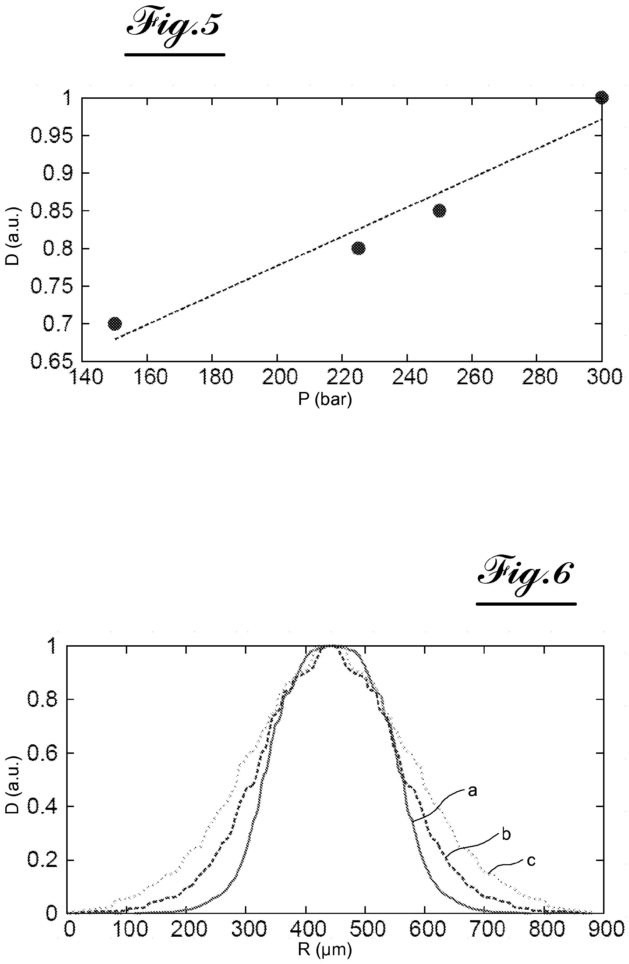

L'utilisation de l'agencement générateur de jet de fluide, équipée d'une tuyère de sortie présente encore l'avantage considérable que la largeur du jet et la densité de celui-ci sont indépendantes. Les figures 5 et 6 permettent d'expliquer cette indépendance.

La figure 5 montre la densité D en fonction de la pression P. Cette dernière est indiquée en bar tandis que la densité est normalisée. Les points sont des valeurs qui ont été mesurées. L'évolution de la densité par rapport à la pression peut être illustrée sous forme d'une ligne droite indiquant que la densité augmente progressivement avec la pression.

La figure 6 illustre pour plusieurs hauteurs, c'est-à-dire distances de la sortie de la tuyère, la diminution de la densité d'un jet à partir de son axe longitudinal, perpendiculairement à celui-ci. Les courbes a, b et c sont faites pour des hauteurs respectivement de 100 pm, de 200 pm et de 300 pm. L'ordonnée indique la densité D sous forme de valeurs normalisées, c'est-à-dire chaque fois par rapport à la densité maximale. L'abscisse définit les emplacements R dans la direction perpendiculaire au jet, en micromètres. La figure 5 montre que l'on peut faire varier continuellement la densité de crête en faisant varier la pression et il ressort de la figure 6 que l'on peut choisir une largeur donnée, c'est-à-dire un rayon, en choisissant la hauteur à laquelle le laser agit sur le faisceau.

Par conséquent, si l'on fixe la hauteur et ainsi la largeur, on peut ensuite librement faire varier la densité, de façon indépendante.

L'ensemble de l'agencement générateur 4 de fluide de forte densité selon l'invention fonctionne sous vide secondaire puisqu'il est enfermé dans l'enceinte étanche 9. L'agencement travaille à des hautes cadences en fonction du débit délivré. La cadence peut-être élevée lorsque le débit est relativement faible et le débit est haut en cas de cadences faibles, ceci en raison du volume de l'enceinte pompée et du rendement des pompes à vide, qui sont avantageusement du type turbo-moléculaire.

Comme il a été indiqué plus haut, la tuyère structure l'écoulement. La taille de celle-ci détermine la taille du plasma. La pression qui alimente la tuyère détermine la densité. Pour une tuyère donnée, on peut donc faire varier indépendamment la densité en faisant varier la pression et la longueur.

Il est à noter que l'impact du faisceau laser se fait à une distance très faible de la sortie de la tuyère dans la zone de la plus forte densité des impulsions de fluide, de l'ordre de 100 à 200 micromètres par exemple. Le faisceau laser sera focalisé sur les impulsions. Etant donné que le faisceau présente ainsi une certaine conicité, avec le diamètre se rétrécissant en direction de l'impact sur l'impulsion, la face frontale 46, au lieu d'être perpendiculaire à l'axe de la sortie, pourrait être légèrement inclinée vers l'arrière à partir de la sortie, d'un angle $\gamma$, afin que le faisceau laser puisse agir sur l'impulsion le plus prêt possible de la sortie de tuyère.

Concernant le fluide utilisé, on emploie avantageusement du gaz, tel que de l'hélium mais l'emploi de n'importe quel autre gaz est envisageable. On pourrait aussi utiliser un liquide qui serait alors adapté pour un couplage efficace entre le plasma et le laser.

Le système ainsi obtenu permet de réaliser un jet de fluide formé par une séquence d'impulsions de haute cadence et d'une forte densité pouvant être supérieure à $10^{19}$ cm$^{-3}$ jusqu'à la densité atomique critique de $10^{21}$ cm$^{-3}$ pour un laser Ti : SAPPHIRE. La cadence peut être supérieure à quelques Hertz, jusqu'à 300 Hertz et même plus. Le gaz peut être fourni par le surpresseur à une pression de 400 bar à température ambiante. Cette pression pourrait être supérieure et, le cas échéant, aller jusqu'à 1000 bar. Comme il a été précisé plus haut, la pression qui règne à l'intérieur de l'enceinte est de $10^{-7}$ bar. En raison de la courte durée des impulsions créées par la valve et malgré les fortes densités et de la faible masse des impulsions de fluide due aux petites dimensions de la tuyère, il est possible d'obtenir ce vide à l'intérieur de l'enceinte, à l'aide de pompes à vide de type par exemple turbo-moléculaire sans que ces pompes se détériorent. Grâce à des jets de haute cadence et de haute densité que l'agencement selon l'invention permet de produire, l'invention rend possible d'explorer des domaines de densité et de cadence de jet de fluide qui, jusqu'à présent, étaient inexplorables. L'invention ouvre ainsi de multiples applications dont certaines seront décrites ci-après à titre d'exemples.

Ces applications se divisent en deux domaines principaux d'application, à savoir la génération de particules et de rayonnement, d'une part, et la mise en forme de faisceaux laser intenses, d'autre part.

En se référant à la figure schématique 7, on décrit tout d'abord un procédé et un système de production de faisceaux d'ions de spectre fin à haute cadence dans la direction du laser et d'émission d'ions de spectre large à haute cadence dans la direction perpendiculaire au laser. Un spectre est considéré comme étant fin quand la variation de l'énergie $\Delta E$ par rapport à l'énergie moyenne E du spectre est inférieure à 1%.

A cette fin, on utilise un faisceau laser intense de par exemple $10^{17}$ W/cm$^2$ à $10^{22}$ W/cm$^2$, composé d'une ou de plusieurs impulsions courtes d'une durée par pulsions inférieure à quelques picosecondes et d'une durée entre les impulsions de quelques picosecondes. Cette impulsion peut être structurée de différentes manières de façon connue en soi, à l'aide d'un amplificateur final pour des lasers à $CO_2$ qui comportent déjà des impulsions structurées ou, plus généralement, de façon optique en subdivisant une impulsion principale puis en retardant d'un retard voulu chaque sous-impulsion obtenue après division. L'impulsion structurée intense crée un plasma à partir du fluide lors de l'impact avec celle-ci, à savoir elle crée une charge électrique libre, formée par des ions et électrons, par "claquage" du plasma dès que la fluence du laser dépasse environ 1 J/cm$^2$.

Bien entendu, on pourrait aussi utiliser une seule impulsion à la place d'une impulsion structurée formée par plusieurs sous-impulsions. L'intérêt d'utiliser une impulsion structurée plutôt qu'une seule impulsion est de faciliter l'enfoncement de la cible par un laser de puissance moyenne identique, mais de puissance de crête inférieure. En effet, chaque impulsion agit comme un piston grâce à la pression radiative qu'il exerce sur le plasma qui est ainsi foré par à-coups, cette force étant proportionnelle à l'intensité divisée par la vitesse de la lumière. Comme avec un marteau et un clou, il est plus facile de percer la matière par ce dispositif séquentiel. Cela n'est pas indispensable mais simplement plus économique en terme de puissance moyenne par impulsion laser.

Ce perçage efficace permet au laser de transférer son énergie sur tout le volume aux électrons du plasma qui chauffent et, par la physique des plasmas, permettent de créer un faisceau d'ions possédant un spectre fin où tous les ions ont quasiment la même énergie à quelques pourcentages près, orientés dans la direction de propagation du laser. Il existe aussi une émission d'ions principalement dans la direction perpendiculaire à la propagation du laser, mais ce n'est pas sous forme d'un faisceau directif et le spectre émis est large.

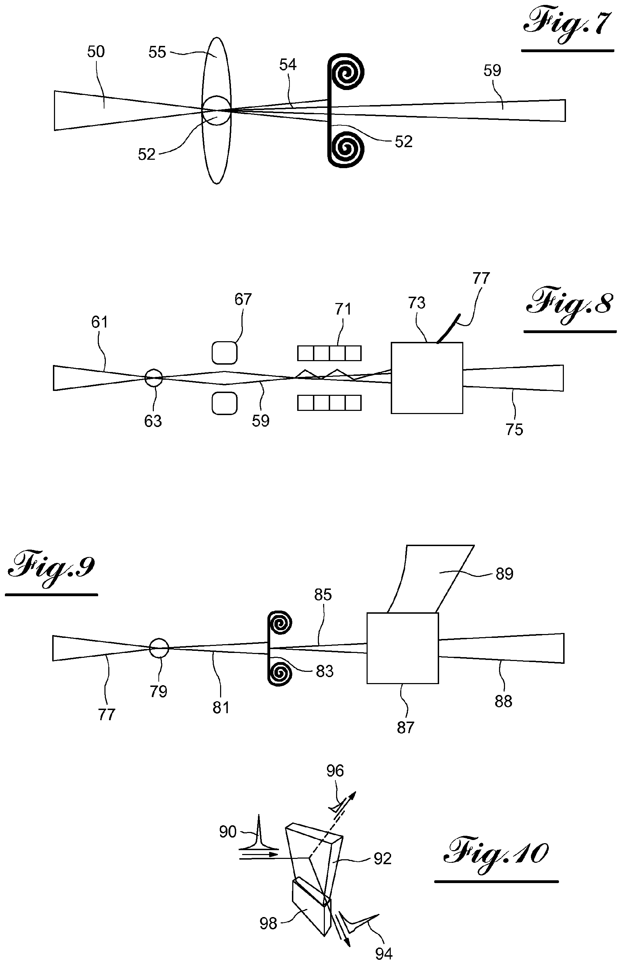

On peut donc envisager un dispositif selon la figure 7, comprenant un laser avec une impulsion structurée ou pas, focalisé sur un jet de fluide à haute cadence et de densité surcritique pour ce laser. Dans le cas d'un laser $CO_2$, la densité est de $10^{19}$ cm$^{-3}$, pour un laser Ti : SAPPHIRE la densité serait de $10^{21}$ cm$^{-3}$. Sur la figure 7, le faisceau laser structuré focalisé est indiqué en 50. L'impact du laser sur le jet de fluide haute cadence indiqué en 52 crée un faisceau d'ions 54 spectralement fin à haute cadence dans l'axe du laser et provoque une émission spectralement large 55 dans la direction perpendiculaire.

Afin de filtrer le laser, on utilise un filtre circulant 57, par exemple une feuille en aluminium de quelques dizaines à centaines de micromètres d'épaisseur, qui défile à une vitesse de 1cm/s ou plus, ce qui permet de rafraîchir la surface que le laser endommage à chaque impact. La figure 7 montre le faisceau d'ions haute cadence, de spectre fin, ainsi obtenu en 59.

Ce faisceau peut être utilisé pour déposer de la dose, c'est-à-dire de l'énergie dans la matière à une épaisseur donnée du fait que le spectre est fin. L'émission large 55 peut servir à sonder des régions de champ électrique fort comme notre plasma. On obtient une carte codée en contraste de gradients de champ.

La figure 8 illustre une autre application de l'invention, à savoir la production de faisceaux X à haute cadence par injection d'un faisceau d'électrons à haute cadence refocalisé dans un onduleur.

Une impulsion laser intense notée 61 est focalisée dans un jet 63 de gaz haute cadence et de densité d'environ $10^{19}$ atomes/cm$^3$. On utilise une optique de focalisation de focal, adapté à la longueur du jet pour que le laser s'y propage de façon régulière, sans filamentation, c'est-à-dire sans exploser à la manière d'un éclair qui se ramifie. Les expériences en laboratoires ont montré que certains électrons, sur le trajet du laser, peuvent être "piégés" par le champ du laser, c'est-à-dire ils suivent le laser, et être accélérés à de hautes énergies tout au long de la propagation. L'énergie finale des électrons dépend de la zone du jet où les électrons sont injectés pour être piégés dans le sillage du laser. L'énergie des électrons sera élevée lorsque la zone d'injection est localisée à l'entrée du jet et relativement plus faible dans le cas de la localisation au niveau de la sortie. Les électrons émergent du plasma sous forme d'un faisceau 65 légèrement divergeant et courent dans le temps, de durée dépendant de celle de l'impulsion laser. Le faisceau haute cadence 65 est spatialement refocalisé au moyen d'un aimant quadripolaire 67 ou de plusieurs aimants quadripolaires montés en série. L'utilisation de plusieurs quadripoles permet d'opérer séquentiellement les corrections de trajectoire des électrons à refocaliser, ce qui est techniquement plus simple à exécuter et génère moins d'erreurs qu'avec un seul aimant quadripolaire plus puissant. La valeur des champs magnétiques des aimants est liée à la dispersion et à l'énergie des électrons du faisceau incident. Le faisceau d'électrons refocalisé et donc convergeant, noté 69 est injecté dans un onduleur 71. Celui-ci est constitué d'une succession d'aimants de polarités alternées de sorte que les électrons "ondulent" au passage et rayonnent des rayons X vers l'avant selon le principe physique de l'émission radiative par un électron accéléré. La période des aimants alternés et leur puissance définissent la gamme spectrale de l'émission X. Si les aimants sont ajustés en accord avec l'énergie d'injection des électrons, la puissance de l'émission X croît à mesure que les électrons avancent en ondulant dans l'onduleur et le spectre X obtenu est fin, c'est-à-dire il y a accord de la phase et émission constructive. Un aimant bipolaire 73 est placé en sortie de l'onduleur pour dévier les électrons et ne conserve que le faisceau de rayons X. Ce dernier est indiqué par la référence 75 tandis que le faisceau d'électrons dévié est indiqué en 77. Le faisceau 75 est un faisceau de particules X haute cadence et constitue un laser X pulsé.

Le dispositif selon la figue 8 permet d'ajuster en variant la densité du jet de gaz, l'énergie des électrons pour avoir un accord optimal avec les caractéristiques des aimants quadripolaires et de l'onduleur.

Par conséquent, la figure 8 illustre l'implémentation d'un laser X pulsé à partir de l'interaction d'un laser intense 61 et d'un jet haute cadence 63.

La figure 9 montre une troisième application de l'invention, à savoir un système de production de faisceaux gamma à haute cadence par conversion d'un faisceau d'électrons à haute cadence par un convertisseur circulant.

Dans cette application, une impulsion laser intense 77 est focalisée dans un jet de gaz 79 de haute cadence et de densité d'environ $10^{19}$ atomes/cm$^3$. Pour focaliser le laser, on utilise une optique de focalisation de focal adaptée à la longueur du jet pour que le laser s'y propage de façon régulière, sans filamentation, c'est-à-dire sans exploser à la manière d'un éclair qui se ramifie. Comme cela vient d'être expliqué dans le cadre de la seconde application, les expériences en laboratoire ont montré que certains électrons sur le trajet du laser peuvent être "piégés" par le champ du laser et être accélérés à de hautes énergies tout au long de la propagation. Comme indiqué plus haut, l'énergie finale des électrons dépend de la zone du jet où les électrons sont injectés pour être pigés dans le sillage du laser. Les électrons émergent du plasma sous forme d'un faisceau 81 faiblement divergeant et court dans le temps, d'une durée dépendant de celle de l'impulsion laser. Le faisceau d'électrons comporte un convertisseur circulant 83, par exemple typiquement une feuille de tantale de 1 mm d'épaisseur, qui défile à une vitesse de 1 cm/s ou plus, ce qui permet de rafraîchir la surface que le laser endommage à chaque impact. En pénétrant dans le convertisseur, les électrons sont freinés par collision sur les atomes, ce qui entraîne une émission de rayons gamma sous forme de faisceaux vers l'avant, par un effet connu sous le terme "bremsstrahlumg". On obtient ainsi un faisceau de rayons gamma à haute cadence noté 85. Ce faisceau des électrons ayant traversé le convertisseur entre dans un aimant bipolaire 87 qui permet l'obtention d'un faisceau de rayons gamma à haute cadence 88 et fait dévier des électrons sous forme d'un faisceau d'électrons dévié 89.

Le dispositif selon la figure 9 permet donc d'obtenir un faisceau de rayons gamma à haute cadence. Pour une position donnée du convertisseur circulant 83 par rapport au jet 79, une variation de l'épaisseur du convertisseur permet de faire varier le nombre de rayons gamma obtenus en sortie, c'est-à-dire la brillance de la source gamma, et la taille de la source. Ces rayons gamma permettent de sonder de la matière dense et d'obtenir par contraste d'absorption des cartes radiographiques pouvant servir à faire la tomographie d'un objet avec une résolution typique de 50 pm.

On décrira ci-après une quatrième application concernant le deuxième domaine de la mise en forme des impulsions laser intenses. Cette application préconise le nettoyage temporel des impulsions intenses par induction d'un miroir plasma conformément à la figure 10.

Dans une impulsion laser intense, l'impulsion principale est précédée de lumière parasite (émission spontanée amplifiée ou ASE en anglais) qui peut être gênante pour les utilisations d'interaction lorsque son intensité est modérée car elle peut entraîner un claquage prématuré de la matière avec laquelle on désire réaliser l'interaction, ce qui rend la structure de la cible indéterminée. Cette lumière parasite est de même longueur d'onde que l'impulsion principale et de même polarisation. La sélection optique n'est donc pas aisée.

Par contre, on peut discriminer la lumière parasite par son intensité qui est beaucoup plus faible que celle de l'impulsion principale.

Comme le montre la figure 10, l'impulsion laser à filtrer, indiquée par la référence 90, est incidente sur un jet de fluide 92 à densité sur-critique pour la longueur d'onde du laser utilisé. Le jet 92 est translucide à l'impulsion tant que le claquage du plasma n'est pas intervenu. Ainsi, tant que la fluence du laser (énergie par surface du faisceau laser) n'atteint pas 1 J/cm$^2$ ou, ce qui est équivalent pour une donnée d'impulsion donnée, que l'intensité n'atteint pas 10$^{10}$ W/cm$^2$, le jet de fluide reste transparent et l'impulsion passe à travers. Dès que ces limites sont franchies, le fluide claque et il se développe un plasma sur critique à la surface, dans lequel le laser ne peut plus pénétrer. Le restant de l'impulsion est donc réfléchi.

La figure 10 montre en 94 l'impulsion laser réfléchie et en 96 l'impulsion laser qui traverse le jet de fluide 92 sortant de la tuyère notée 98.

Dans cette application, le jet de fluide 92 à haute densité, notamment liquide, et haute cadence, fonctionne donc comme un miroir sélectif qui absorbe les intensités laser modérées parasites au début de l'impulsion incidente 90 et réfléchit les intensités fortes sous forme d'une impulsion 94 qui pourrait servir à produire une interaction dans des conditions plus contrôlées.

Une cinquième application concernant ce domaine de mise en forme des impulsions laser intenses assure une compression temporelle et spatiale des impulsions.

Dans cette application, une impulsion laser intense est focalisée dans le jet de gaz haute cadence et de densité d'environ 10$^{19}$ atomes/cm$^3$. On utilise une optique de focalisation de focal adaptée à la longueur du jet pour que le laser s'y propage de façon régulière, sans filamentation.

Cette impulsion est en fait composée de plusieurs longueurs d'onde de déphasages relatifs. Or, le plasma a pour effet d'induire des retards de phase différents suivant la longueur d'onde. Les petites longueurs d'onde voyagent moins vite dans le plasma que les grandes longueurs d'onde. Par conséquent, si le déphasage relatif compris entre deux longueurs d'ondes différentes de l'impulsion laser à l'entrée du plasma est judicieusement choisi, c'est-à-dire si les petites longueurs d'onde arrivent sur le plasma avant les grandes longueur d'onde (on dit qu'on dérive les fréquences positivement) d'une façon que le parcours dans le plasma puisse resynchroniser les composantes, à savoir remettre en phase les composantes spectrales, le plasma peut contribuer à recomprimer l'impulsion dans le temps. La dérive de fréquence peut être obtenue de façon connue en soi, par exemple à l'aide d'un dispositif commercialisé sous la dénomination DAZZLER.

La recompression dans le temps par mise en phase des composantes du spectre vient directement de la relation de Fourier dT·dW = constante. Ainsi, un large contenu spectral en phase dW d'une impulsion donne une faible durée dT à l'impulsion. On peut donc obtenir en sortie une impulsion plus courte dans le temps et plus intense si le plasma n'a pas absorbé trop d'énergie. Il est à noter que ce principe est largement expliqué dans la littérature. Ce type de compression est jusqu'à présent réalisé par des réseaux qui s'usent avec le temps et ne supporte que des cadences inférieures de quelques Hz et présente une efficacité en énergie de 60 % (taux d'énergie transmise). Le dispositif selon l'invention permet d'obtenir une compression avec un jet de fluide à haute cadence avec une efficacité en énergie de 20% environ. De même, le plasma formé par l'impulsion intense peut entraîner la focalisation spatiale à l'impulsion par effet Kerr et donc intensifier l'impulsion en sortie du jet.

Il vient d'être démontré que l'invention permet d'atteindre des densités atomiques supérieures à 10$^{21}$ atomes/cm$^3$. Grâce à cette caractéristique, l'invention permet d'atteindre ce qu'il est appelé la densité plasma critique à partir de laquelle un laser de puissance qui ionise le gaz du jet sortant de la tuyère en plasma, ne peut plus se propager. Cette densité critique est donnée par l'expression :

$$n_c \ [cm^{-3}] \sim 1,1 \ \times \ 10^{21}/L^2 \ [\mu m]$$

où L est la longueur d'onde du laser utilisée.

Par conséquent l'invention permet d'obtenir la densité critique atomique pour tout laser de puissance de longueur d'onde supérieure à 0,750 micromètres. En particulier, on peut atteindre la densité critique pour des lasers Titane : SAPPHIRE (L = 0,81 pm) qui constitue la grande majorité des lasers de puissance actuellement utilisée en science. Ceci présente un intérêt fort car, à partir de cette densité, l'interaction laser-plasma assure un couplage très efficace, à savoir une conversion presque totale de l'énergie laser en énergie interne du plasma.

D'autre part, l'invention permet d'obtenir une compression temporelle non-linéaire des impulsions laser intenses. A cette fin une impulsion laser intense est focalisée dans le jet de gaz haute cadence d'une densité d'environ $10^{19}$ atomes / $cm^3$. On utilise une optique de focalisation adaptée à la longueur du jet pour que le laser s'y propage de façon régulière, sans filamentation. Lorsque l'impulsion intense traverse le jet de gaz, elle modifie directement l'indice optique du plasma, d'une part, et d'autre part, génère une vague plasma d'amplitude très élevée qui se déplace avec l'impulsion. Ces deux effets non-linéaires agissent en retour sur l'impulsion dans le domaine fréquentiel, ce qui a pour conséquence dans le domaine temporel de faire voyager l'arrière de l'impulsion plus vite que l'avant, et aboutit à la compression de l'impulsion. Par ce processus, l'impulsion courte et intense peut être comprimée dans le temps par exemple, d'un facteur 3 sur quelques millimètres de gaz, et donc produire des impulsions ultra-intenses sans risque de destruction de la structure qui compresse. L'utilisation d'un jet à haute cadence et haute densité peut rendre ce processus réalisable à haute cadence sur une centaine de micromètres seulement, ce qui constitue une alternative aux techniques actuellement en usage pour la mise en forme des lasers intenses à haute cadence.

## Revendications

1. Procédé pour générer un jet de fluide de forte densité atomique, **caractérisé en ce qu'**il comprend l'opération de créer à l'aide d'une électrovanne rapide haute pression (5), de l'ordre de 400 bars et plus, suivie d'une tuyère (7) montée sur l'ouverture de sortie de l'électrovanne, un jet de fluide pulsé (F) de dimensions submillimétriques et d'une densité atomique supérieure à $10^{20}$ $cm^{-3}$.

2. Procédé selon la revendication 1, **caractérisé en ce que** la densité atomique du jet de fluide présente une valeur de $10^{21}$ $cm^{-3}$ ou plus.

3. Procédé selon l'une des revendications 1 ou 2, **caractérisé en ce que** le fluide est un gaz tel que de l'hélium.

4. Procédé selon l'une des revendications 1 ou 2, **caractérisé en ce que** le fluide est biphasique, tel qu'un jet d'agrégat.

5. Procédé selon l'une des revendications 1 à 2, **caractérisé en ce que** le fluide est un liquide tel que de l'eau.

6. Agencement générateur d'un jet de fluide de forte densité atomique, pour la mise en œuvre du procédé selon l'une des revendications 1 à 5, comprenant une source de fluide sous pression (1, 2), **caractérisé en ce qu'**il comprend en outre une électrovanne (5) haute pression, de l'ordre de 400 bar et plus, d'ouverture et de fermeture rapide du canal (16) de passage du fluide à travers l'électrovanne, et une tuyère (7) de dimensions submillimétriques adaptée pour accélérer et structurer le jet de fluide pulsé de dimensions submillimétriques, produit par l'électrovanne (5).

7. Agencement selon la revendication 6, **caractérisé en ce que** l'électrovanne (5) comporte un solénoïde (18) et un élément mobile d'ouverture et de fermeture rapide du canal (16) de passage du fluide à travers l'électrovanne, qui est déplaçable perpendiculairement à ce canal, dans sa position d'ouverture du canal, sous l'effet du champ magnétique produit par le solénoïde et dont le retour dans sa position de fermeture du canal se produit sous l'effet du fluide sous pression.

8. Agencement selon la revendication 7, **caractérisé en ce que** l'entrefer (20) autour du canal (16) comporte au niveau de l'élément mobile (24) une asymétrie ayant pour conséquence la création d'une composante de champ magnétique orientée perpendiculairement au canal et destinée à provoquer le déplacement de l'élément mobile (24) vers sa position d'ouverture du canal (16).

9. Procédé pour créer un plasma, **caractérisé en ce que** l'on utilise un jet de fluide obtenu selon l'une des revendications 1 à 5 et **en ce que** l'on fait agir sur le jet (F) sortant de la tuyère (7) un laser (L) dont le faisceau est orienté perpendiculairement au jet, à l'intérieur d'une enceinte à vide (9).

10. Procédé selon la revendication 9, **caractérisé en ce que** l'on crée un jet de fluide pulsé d'une forte densité atomique, mais d'une largeur suffisamment faible pour que l'on puisse utiliser pour la création du vide dans l'enceinte (9) une pompe à très haute vitesse, telle qu'une pompe de type turbo-moléculaire.

11. Procédé selon l'une des revendications 9 ou 10, **caractérisé en ce que** l'on crée un vide de l'ordre de $10^{-7}$ bar à l'intérieur de l'enceinte (9).

12. Système générateur d'un plasma pour la mise en œuvre du procédé selon l'une des revendications 9 à 11, **caractérisé en ce qu'**il comprend une source d'un fluide sous pression, un agencement (4) générateur d'un jet de fluide d'une pression de l'ordre de 400 bar et plus selon l'une des revendications 6 ou 7, un laser (L) dont le faisceau agit sur le jet de fluide (F) à la sortie de la tuyère (7), une enceinte à vide (9) dans laquelle est disposé l'agencement générateur du jet et dans lequel a lieu l'impact du laser (L) sur le jet de fluide (F) et **en ce qu'**il comporte une pompe (10) de création du vide à l'intérieur de l'enceinte (9), qui est du type à très haute vitesse telle qu'une pompe turbo-moléculaire.

**13.** Procédé d'application du système selon la revendication 12, **caractérisé en ce que** le système est utilisé pour produire des faisceaux d'ions (59) de spectre fin où tous les ions ont approximativement la même énergie et à haute cadence dans la direction du laser et d'émission d'ions (55) de spectre large à haute cadence dans la direction perpendiculaire au laser.

**14.** Procédé selon la revendication 13, **caractérisé en ce que** l'on utilise un faisceau laser intense (50) composé d'une ou de plusieurs impulsions courtes de l'ordre de quelques picosecondes et d'un écart entre les impulsions de quelques picosecondes, focalise ce faisceau laser sur un jet de fluide (52) à haute cadence et de densité sur-critique pour ce laser.

**15.** Procédé selon la revendication 14, **caractérisé en ce que** l'on filtre le faisceau d'ions (54) à haute cadence sortant du plasma dans l'axe laser en utilisant avantageusement un filtre mobile (57) tel qu'une feuille en aluminium de quelques dizaines à centaines de micromètres pour permettre de rafraîchir la surface que le faisceau endommage à chaque impact.

**16.** Procédé d'application du système selon la revendication 12, **caractérisé en ce que** l'on utilise le système pour produire un faisceau (X) à haute cadence.

**17.** Procédé selon la revendication 16, **caractérisé en ce que** l'on focalise une impulsion laser intense (61) dans un jet de fluide (63) haute cadence et de forte densité telle qu'une densité de l'ordre de $10^{19}$ atomes/cm$^3$ pour produire un faisceau d'électrons faiblement divergent (65) et d'une courte durée, refocalise spatialement ce faisceau et fait passer le faisceau refocalisé à travers un onduleur (71).

**18.** Procédé selon la revendication 17, **caractérisé en ce que** l'on fait passer le faisceau ayant traversé l'onduleur (71) par un dispositif (73) qui fait dévier les électrons et permet d'obtenir un faisceau seulement de rayons X (75).

**19.** Procédé selon l'une des revendications 17 ou 18, **caractérisé en ce qu'**on utilise un onduleur (71) constitué d'une succession d'aimants de polarité alternés de sorte que les électrons ondulent au passage de l'onduleur et rayonnent des rayons X vers l'avant dans la direction du faisceau.

**20.** Procédé selon la revendication 19, **caractérisé en ce que** l'on fait varier la gamme spectrale des rayons X par variation de la période des aimants alternés et leur puissance.

**21.** Procédé d'application du système selon la revendication 12, **caractérisé en ce que** l'on utilise ce système pour la production de faisceaux gamma (88) à haute cadence.

**22.** Procédé selon la revendication 21, **caractérisé en ce que** l'on produit les faisceaux gamma (88) à haute cadence par conversion d'un faisceau d'électrons (81) à haute cadence, obtenu à partir du plasma produit par interaction d'une impulsion laser intense (77) avec un jet de gaz (79) haute cadence et de forte densité telle qu'une densité de l'ordre de $10^{19}$ atomes/cm$^3$.

**23.** Procédé selon la revendication 22, **caractérisé en ce que** l'on focalise l'impulsion laser intense (77) dans le jet de gaz (79) et utilise une optique de focalisation de focal adapté à la longueur du jet pour que le laser s'y propage de façon régulière, sans filamentation.

**24.** Procédé selon l'une des revendications 22 ou 23, **caractérisé en ce que** l'on fait impacter le faisceau d'électrons (81) un convertisseur mobile (83) pour provoquer l'émission de rayons gamma dus au freinage des électrons par collision sur les atomes du convertisseur.

**25.** Procédé selon la revendication 24, **caractérisé en ce que** l'on utilise un dispositif tel qu'un aimant bipolaire (85) pour faire dévier les électrons et l'obtention d'un faisceau de rayons gamma à haute cadence (87) .

**26.** Procédé d'application du système selon la revendication 12, **caractérisé en ce que** le système est utilisé pour réaliser un nettoyage temporel des impulsions intenses (90) du laser à l'aide du jet de fluide (92).

**27.** Procédé selon la revendication 26, **caractérisé en ce que** l'impulsion laser (90) à nettoyer est amenée à être incidente sur un jet de fluide (92) à densité sur-critique pour la longueur d'onde du laser de façon que la lumière parasite de l'impulsion ne produise pas un claquage du plasma et soit translucide à cette lumière et que le claquage ne soit produit que par la partie utile de l'impulsion laser qui est alors réfléchie par le jet de fluide.

**28.** Procédé d'application du système selon la revendication 12, **caractérisé en ce que** l'on utilise ce système pour réaliser une compression temporelle et spatiale des impulsions laser (90).

**29.** Procédé selon la revendication 28, **caractérisé en ce qu'**une impulsion laser intense à comprimer est focalisée dans le jet de gaz haute cadence et de forte densité telle qu'une densité de l'ordre de $10^{19}$ ato-

mes/cm³ et fait en sorte que les petites longueurs d'onde du spectre de l'impulsion arrivent sur le plasma avant les grandes longueurs d'onde de façon que la propagation moins vite des petites longueurs d'onde dans le plasma provoque une recompression de l'impulsion dans le temps.

**Patentansprüche**

1. Verfahren zum Erzeugen eines Fluidstrahls mit hoher Atomdichte, **dadurch gekennzeichnet, dass** es den Vorgang des Herstellens, mit Hilfe einer schnellen Hochdruck-Magnetventils (5) im Bereich von 400 bar und mehr, gefolgt von einer Düse (7), die auf der Ausgangsöffnung des Magnetventils montiert ist, eines gepulsten Fluidstrahls (F) mit submillimetrischen Abmessungen und einer Atomdichte von mehr als $10^{20}$ cm$^{-3}$, umfasst

2. Verfahren nach Anspruch 1, **dadurch gekennzeichnet, dass** die Atomdichte des Fluidstrahls einen Wert von $10^{21}$ cm$^{-3}$ oder mehr aufweist.

3. Verfahren nach einem der Ansprüche 1 oder 2, **dadurch gekennzeichnet, dass** das Fluid ein Gas wie z. B. Helium ist.

4. Verfahren nach einem der Ansprüche 1 oder 2, **dadurch gekennzeichnet, dass** das Fluid biphasisch ist, wie z. B. ein Aggregatstrahl.

5. Verfahren nach einem der Ansprüche 1 bis 2, **dadurch gekennzeichnet, dass** das Fluid eine Flüssigkeit wie z. B. Wasser ist.

6. Anordnung zum Erzeugen eines Fluidstrahls mit hoher Atomdichte zur Durchführung des Verfahrens nach einem der Ansprüche 1 bis 5, umfassend eine Fluidquelle unter Druck (1, 2), **dadurch gekennzeichnet, dass** sie außerdem ein Hochdruck-Magnetventil (5) im Bereich von 400 bar und mehr zum schnellen Öffnen und Schließen des Kanals (16) zum Durchgang des Fluids durch das Magnetventil und eine Düse (7) mit submillimetrischen Abmessungen umfasst, die ausgelegt ist, um den gepulsten Fluidstrahl mit submillimetrischen Abmessungen, erzeugt vom Magnetventil (5), zu beschleunigen und zu strukturieren.

7. Anordnung nach Anspruch 6, **dadurch gekennzeichnet, dass** das Magnetventil (5) ein Solenoid (18) und ein mobiles Element zum schnellen Öffnen und Schließen des Kanals (16) zum Durchgang des Fluids durch das Magnetventil umfasst, das senkrecht zu diesem Kanal in seine Position zur Öffnung des Kanals unter der Wirkung des Magnetfelds, erzeugt vom Solenoid, verschoben werden kann, und wobei die Rückkehr in seine Position zum Verschluss des Kanals unter der Wirkung des Fluid unter Druck erfolgt.

8. Anordnung nach Anspruch 7, **dadurch gekennzeichnet, dass** der Luftspalt (20) um den Kanal (16) auf der Ebene des beweglichen Elements (24) eine Asymmetrie umfasst, die als Konsequenz das Erzeugen einer Magnetfeldkomponente aufweist, die senkrecht zum Kanal ausgerichtet und ausgelegt ist, um die Verschiebung des beweglichen Elements (24) in seine Position des Öffnens des Kanals (16) zu verursachen.

9. Verfahren zum Erzeugen eines Plasmas, **dadurch gekennzeichnet, dass** ein Fluidstrahl, erhalten nach einem der Ansprüche 1 bis 5, verwendet wird, und dadurch, dass auf den Strahl (F), der aus der Düse (7) austritt, ein Laser (L) zur Wirkung gebracht wird, dessen Bündel senkrecht zum Strahl ausgerichtet ist, im Inneren eines Vakuumbehälters (9).

10. Verfahren nach Anspruch 9, **dadurch gekennzeichnet, dass** ein gepulster Fluidstrahl mit einer hohen Atomdichte erzeugt wird, jedoch mit einer ausreichend geringen Länge, damit zur Erzeugung des Vakuums im Behälter (9) eine Pumpe mit sehr hoher Geschwindigkeit wie z. B. vom Typ Turbomolekularpumpe verwendet werden kann.

11. Verfahren nach einem der Ansprüche 9 oder 10, **dadurch gekennzeichnet, dass** ein Vakuum im Bereich von $10^{-7}$ im Inneren des Behälters (9) erzeugt wird.

12. System zur Erzeugung eines Plasmas zur Durchführung des Verfahrens nach einem der Ansprüche 9 bis 11, **dadurch gekennzeichnet, dass** es eine Quelle eines Fluids unter Druck, eine Anordnung (4) zur Erzeugung eines Fluidstrahls mit einem Druck im Bereich von 400 bar und mehr nach einem der Ansprüche 6 oder 7, einen Laser (L), dessen Bündel auf den Fluidstrahl (F) am Ausgang der Düse (7) wirkt, einen Behälter unter Vakuum (9), in dem sich die Anordnung zur Erzeugung des Strahls befindet, und in dem der Aufprall des Lasers (L) auf den Fluidstrahl (F) erfolgt, umfasst, und dadurch, dass es eine Pumpe (10) zur Erzeugung des Vakuums im Inneren des Behälters (9) umfasst, die vom Typ mit sehr hoher Geschwindigkeit wie z. B. eine Turbomolekularpumpe ist.

13. Verfahren zur Anwendung des Systems nach Anspruch 12, **dadurch gekennzeichnet, dass** das System verwendet wird, um Ionenbündel (59) mit engem Spektrum, in dem alle Ionen ungefähr die gleiche Energie aufweisen, und mit hohem Takt in der Richtung des Lasers und mit einer Ionenemission (55) mit breitem Spektrum mit hohem Takt in der senkrechten Richtung zum Laser zu erzeugen.

14. Verfahren nach Anspruch 13, **dadurch gekennzeichnet, dass** ein intensives Laserbündel (50) verwendet wird, zusammengesetzt aus einem oder mehreren kurzen Impulsen im Bereich von einigen Pikosekunden und mit einer Beabstandung zwischen den Impulsen von einigen Pikosekunden, dieses Laserbündel auf einen Fluidstrahl (52) mit hohem Takt und überkritischer Dichtheit für diesen Laser fokussiert wird

15. Verfahren nach Anspruch 14, **dadurch gekennzeichnet, dass** dieses Ionenbündel (54) mit hohem Takt, das aus dem Plasma in der Laserachse austritt, vorteilhafterweise unter Verwendung eines beweglichen Filters (57) wie z. B. eines Aluminiumblatts mit einigen Zehntel bis Hundertstel Mikrometer gefiltert wird, um zu ermöglichen, die Fläche, die vom Bündel bei jedem Aufprall beschädigt wird, abzukühlen.

16. Verfahren zur Anwendung des Systems nach Anspruch 12, **dadurch gekennzeichnet, dass** das System verwendet wird, um ein Bündel (X) mit hohem Takt zu erzeugen.

17. Verfahren nach Anspruch 16, **dadurch gekennzeichnet, dass** ein intensiver Laserimpuls (61) in einen Fluidstrahl (63) mit hohem Takt und hoher Dichte wie z. B. einer Dichte im Bereich von $10^{19}$ Atomen/cm$^3$ fokussiert wird, um ein Elektronenbündel (65), das schwach divergierend und von einer kurzen Dauer ist, zu erzeugen, dieses Bündel räumlich neu fokussiert wird und das neu fokussierte Bündel durch einen Wechselrichter (71) geschickt wird.

18. Verfahren nach Anspruch 17, **dadurch gekennzeichnet, dass** das Bündel, das den Wechselrichter (17) durchquert hat, durch eine Vorrichtung (73) geschickt wird, die die Elektronen abweichen lässt und ermöglicht, ein Bündel nur von Röntgenstrahlen (75) zu erhalten.

19. Verfahren nach einem der Ansprüche 17 oder 18, **dadurch gekennzeichnet, dass** ein Wechselrichter (71) verwendet wird, der aus einer Aufeinanderfolge von Magneten mit wechselnden Polaritäten besteht, so dass die Elektronen beim Durchgang durch den Wechselrichter ondulieren und Röntgenstrahlen nach vorne in die Richtung des Bündels strahlen.

20. Verfahren nach Anspruch 19, **dadurch gekennzeichnet, dass** der Spektralbereich der Röntgenstrahlen durch Variation der Periode der wechselnden Magneten und ihrer Stärke variiert wird.

21. Verfahren zur Anwendung des Systems nach Anspruch 12, **dadurch gekennzeichnet, dass** dieses System für die Herstellung von Gammastrahlen (88) mit hohem Takt verwendet wird.

22. Verfahren nach Anspruch 21, **dadurch gekennzeichnet, dass** die Gammastrahlen (88) mit hohem Takt durch Umwandlung eines Elektronenbündels (81) mit hohem Takt erzeugt werden, erhalten aus dem Plasma, das durch die Wechselwirkung zwischen einem intensiven Laserimpuls (77) mit einem Gasstrahl (79) mit hohem Takt und hoher Dichte wie z. B. einer Dichte im Bereich von $10^{19}$ Atomen/cm$^3$ erzeugt wird.

23. Verfahren nach Anspruch 22, **dadurch gekennzeichnet, dass** der intensive Laserimpuls (77) in den Gasstrahl (79) fokussiert wird und eine Fokussierungsoptik mit einem Brennpunkt verwendet wird, die an die Länge des Strahls angepasst ist, damit sich der Laser dort auf regelmäßige Weise ohne Filamentbildung verbreitet.

24. Verfahren nach einem der Ansprüche 22 oder 23, **dadurch gekennzeichnet, dass** das Elektronenbündel (81) auf einen beweglichen Wandler (83) auftreffen gelassen wird, um die Emission von Gammastrahlen aufgrund der Bremsung der Elektronen durch Kollision auf den Atomen des Wandlers hervorzurufen.

25. Verfahren nach Anspruch 24, **dadurch gekennzeichnet, dass** eine Vorrichtung wie z. B. ein bipolarer Magnet (85) verwendet wird, um die Elektronen abweichen zu lassen und ein Bündel von Gammastrahlen mit hohem Takt (87) zu erhalten.

26. Verfahren zur Anwendung des Systems nach Anspruch 12, **dadurch gekennzeichnet, dass** das System verwendet wird, um eine vorübergehende Reinigung der intensiven Impulse (90) des Lasers mit Hilfe des Fluidstrahls (92) durchzuführen.

27. Verfahren nach Anspruch 26, **dadurch gekennzeichnet, dass** der zu reinigende Laserimpuls (90) veranlasst wird, auf einen Fluidstrahl (92) mit überkritischer Dichte für die Wellenlänge des Lasers einzufallen, so dass das Streulicht des Impulses kein Aufbrechen des Plasmas erzeugt und durchlässig für dieses Licht ist, und dass das Aufbrechen nur durch den nützlichen Teil des Laserimpulses erfolgt, der nun durch den Fluidstrahl reflektiert wird.

28. Verfahren zur Anwendung des Systems nach Anspruch 12, **dadurch gekennzeichnet, dass** dieses System verwendet wird, um eine vorübergehende und räumliche Verdichtung der Laserimpulse (90) durchzuführen.

**29.** Verfahren nach Anspruch 28, **dadurch gekennzeichnet, dass** ein intensiver Laserimpuls, der verdichtet werden soll, in den Gasstrahl mit hohem Takt und hoher Dichte, wie z. B. einer Dichte im Bereich von $10^{19}$ Atomen/$cm^3$, fokussiert wird und sich derart verhält, dass die kleinen Wellenlängen des Impulsspektrums das Plasma vor den großen Wellenlängen erreichen, so dass die weniger schnelle Ausbreitung der kleinen Wellenlängen im Plasma eine erneute Verdichtung des Impulses im Laufe der Zeit hervorruft.

## Claims

**1.** A method for generating a jet of fluid with a high atomic density, **characterized in that** it comprises the operation of creating, via a fast high-pressure solenoid valve (5), of the order of 400 bars and more, followed by a nozzle (7) mounted on the outlet opening of the solenoid valve, a pulsed jet of fluid (F) with submillimeter dimensions and an atomic density greater than $10^{20}$ $cm^{-3}$.

**2.** The method according to claim 1, **characterized in that** the atomic density of the jet of fluid has a value of $10^{21}$ $cm^{-3}$ or more.

**3.** The method according to one of claims 1 or 2, **characterized in that** the fluid is a gas such as helium.

**4.** The method according to one of claims 1 or 2, **characterized in that** the fluid is biphasic, such as an aggregate jet.

**5.** The method according to one of claims 1 to 2, **characterized in that** the fluid is a liquid such as water.

**6.** An arrangement generating a jet of atomic high-density fluid, for implementing the method according to one of claims 1 to 5, comprising a source of pressurized fluid (1, 2), **characterized in that** it further comprises a high-pressure solenoid valve (5), of the order of 400 bars and more, with fast opening and closing of the channel (16) for the passage of the fluid through the solenoid valve, and a nozzle (7) with submillimeter dimensions configured to accelerate and structure the pulsed fluid jet with submillimeter dimensions, produced by the solenoid valve (5).

**7.** The arrangement according to claim 6, **characterized in that** the solenoid valve (5) includes a solenoid (18) and a moving element for fast opening and closing of the channel (16) for passage of the fluid through the solenoid valve, which can be moved perpendicular to this channel, in its opening position of the channel, under the effect of the magnetic field produced by the solenoid and the return of which to its closed position of the channel occurs under the effect of the pressurized fluid.

**8.** The arrangement according to claim 7, **characterized in that** the air gap (20) around the channel (16) includes, at the moving element (24), an asymmetry resulting in creating a magnetic field component oriented perpendicular to the channel and configured to cause the movement of the moving element (24) toward its open position of the channel (16).

**9.** A method for creating a plasma, **characterized in that** a fluid jet is used obtained according to one of claims 1 to 5 and **in that** a laser (L) is made to act on the jet (F) leaving the nozzle (7), the beam of said laser being oriented perpendicular to the jet, inside a vacuum chamber (9).

**10.** The method according to claim 9, **characterized in that** a pulsed fluid jet is created with a high atomic density, but a small enough width for it to be possible to use a very high-speed pump, such as a pump of the turbo-molecular type, to create the vacuum in the chamber (9).

**11.** The method according to one of claims 9 or 10, **characterized in that** a vacuum is created of the order of $10^{-7}$ bars inside the chamber (9).

**12.** A system for generating a plasma for carrying out the method according to one of claims 9 to 11, **characterized in that** it comprises a source of a pressurized fluid, an arrangement (4) generating a jet of fluid with a pressure of the order of 400 bars and more according to one of claims 6 or 7, a laser (L) whose beam acts on the jet of fluid (F) at the outlet of the nozzle (7), a vacuum chamber (9) in which the arrangement generating the jet is arranged and in which the impact of the laser (L) takes place on the jet of fluid (F) and **in that** it includes a pump (10) for creating a vacuum inside the chamber (9), which is of the very high-speed type, such as a turbo-molecular pump.

**13.** A method for applying the system according to claim 12, **characterized in that** the system is used to produce ion beams (59) with a fine spectrum where all of the ions have approximately the same energy and a high rate in the direction of the laser and for emitting ions (55) with a broad spectrum and with a high rate in the direction perpendicular to the laser.

**14.** The method according to claim 13, **characterized in that** an intense laser beam (50) is used made up of one or several short pulses of the order of several picoseconds and a gap between the pulses of several picoseconds, focuses this laser beam on a fluid jet (52) with a high rate and supercritical density for this laser.

15. The method according to claim 14, **characterized in that** the ion beam (54) with a high rate leaving the plasma in the laser axis is filtered advantageously using a moving filter (57) such as an aluminum sheet from several tens to several hundreds of micrometers in order to make it possible to refresh the surface that the beam damages upon each impact.

16. A method for applying the system according to claim 12, **characterized in that** the system is used to produce a beam (X) with a high rate.

17. The method according to claim 16, **characterized in that** an intense laser pulse (61) is focused in a jet of fluid (63) with a high rate and high density such as a density of the order of $10^{19}$ atoms/cm$^3$ in order to produce a weakly divergent electron beam (65) with a short duration, spatially refocuses this beam and causes the refocused beam to pass through an inverter (71).

18. The method according to claim 17, **characterized in that** the beam having passed through the inverter (71) is made to pass through a device (73) that deflects the electrons and makes it possible to obtain a beam only with x-rays (75).

19. The method according to one of claims 17 or 18, **characterized in that** an inverter (71) is used made up of a series of magnets of alternating polarity such that the electrons undulate upon passage of the inverter and radiate x-rays forward in the direction of the beam.

20. The method according to claim 19, **characterized in that** the spectral range of the x-rays is varied by variation of the period of the alternating magnets and their power.

21. A method for applying the system according to claim 12, **characterized in that** this system is used to produce gamma beams (88) at a high rate.

22. The method according to claim 21, **characterized in that** the gamma beams (88) are produced at a high rate by converting an electron beam (81) at a high rate, obtained from plasma produced by interaction of an intense laser pulse (77) with a jet of gas (79) at a high rate and a high density such as a density of the order of $10^{19}$ atoms/cm$^3$.

23. The method according to claim 22, **characterized in that** the intense laser pulse (77) is focused in the jet of gas (79) and uses a focusing optic with focal length adapted to the length of the jet so that the laser propagates therein regularly, without filamentation.

24. The method according to one of claims 22 or 23, **characterized in that** the electron beam (81) is made to strike a moving converter (83) to cause the emission of gamma rays due to the slowing of the electrons by collision on the atoms of the converter.

25. The method according to claim 24, **characterized in that** a device is used such as a bipolar magnet (85) to deflect the electrons and obtain a beam of gamma rays at a high rate (87).

26. A method for applying the system according to claim 12, **characterized in that** the system is used to perform a temporal cleaning of the intense pulses (90) of the laser using the jet of fluid (92).

27. The method according to claim 26, **characterized in that** the laser pulse (90) to be cleaned is made to be incident on a jet of fluid (92) with supercritical density for the wavelength of the laser such that the stray light of the pulse does not produce a breakdown of the plasma and is translucent to this light and that the breakdown is only produced by the useful part of the laser pulse, which is then reflected by the jet of fluid.

28. A method for application of the system according to claim 12, **characterized in that** this system is used to produce a temporal and spatial compression of the laser pulses (90).

29. The method according to claim 28, **characterized in that** an intense laser pulse to be compressed is focused in the jet of gas at a high rate and high density such as a density of the order of $10^{19}$ atoms/cm$^3$ and causes the short wavelengths of the spectrum of the pulse to arrive on the plasma before the large wavelengths such that the slower propagation of the short wavelengths in the plasma causes a recompression of the pulse over time.

*Fig.1*

*Fig.2A*

Fig.2B

# *Fig.3A*

# *Fig.3B*

# *Fig.4A*

# *Fig.4C*

# *Fig.4B*

*Fig.5*

*Fig.6*

*Fig.7*

*Fig.8*

*Fig.9*

*Fig.10*

**RÉFÉRENCES CITÉES DANS LA DESCRIPTION**

**Documents brevets cités dans la description**

* US 5577092 A